(19) Europäisches Patentamt
European Patent Office
Office européen des brevets

(11) EP 1 309 355 B1

(12) **EUROPÄISCHE PATENTSCHRIFT**

(45) Veröffentlichungstag und Bekanntmachung des Hinweises auf die Patenterteilung:
**14.05.2008 Patentblatt 2008/20**

(21) Anmeldenummer: **01960612.8**

(22) Anmeldetag: **02.08.2001**

(51) Int Cl.:
***A61K 47/48*** *(2006.01)* ***A61F 2/02*** *(2006.01)*

(86) Internationale Anmeldenummer:
**PCT/EP2001/008932**

(87) Internationale Veröffentlichungsnummer:
**WO 2002/013872 (21.02.2002 Gazette 2002/08)**

(54) **PEPTID- UND PEPTIDMIMETIKAKONJUGATE MIT INTEGRIN-INHIBITOR-EIGENSCHAFTEN**

PEPTIDE AND PEPTIDE MIMETIC CONJUGATES WITH INTEGRIN-INHIBITOR PROPERTIES

CONJUGUES PEPTIDIQUES ET CONJUGUES MIMETIQUES PEPTIDIQUES AYANT DES PROPRIETES D'INHIBITION D'INTEGRINE

(84) Benannte Vertragsstaaten:
**AT BE CH CY DE DK ES FI FR GB GR IE IT LI LU MC NL PT SE TR**

(30) Priorität: **17.08.2000 DE 10040105**

(43) Veröffentlichungstag der Anmeldung:
**14.05.2003 Patentblatt 2003/20**

(73) Patentinhaber: **Biomet Deutschland GmbH
14167 Berlin (DE)**

(72) Erfinder:
- **MEYER, Jörg
63150 Heusenstamm (DE)**
- **NIES, Berthold
64407 Fränkisch-Crumbach (DE)**
- **DARD, Michel
64342 Seeheim-Jugenheim (DE)**
- **HÖLZEMANN, Günter
64342 Seeheim-Jugenheim (DE)**
- **KESSLER, Horst
65842 Schwalbach (DE)**
- **KANTLEHNER, Martin
85354 Freising (DE)**
- **HERSEL, Ulrich
97250 Erlabrunn (DE)**
- **GIBSON, Christoph
79241 Ihringen (DE)**
- **SULYOK, Gabor
80807 München (DE)**

(74) Vertreter: **Gross, Felix et al
Maikowski & Ninnemann
European Patent and Trademark Attorneys
Kurfürstendamm 54-55
10707 Berlin (DE)**

(56) Entgegenhaltungen:
**DE-A- 19 755 801**

- **NAKHLE B M ET AL: "Synthesis of 3,5-Bis (phosphonomethyl)benzoic Acid and Its Application as a Metal Oxide Surface Bivalent Anchor" TETRAHEDRON, ELSEVIER SCIENCE PUBLISHERS, AMSTERDAM, NL, Bd. 55, Nr. 10, 5. März 1999 (1999-03-05), Seiten 2835-2846, XP004157121 ISSN: 0040-4020**
- **CHU ET AL: "Presentation of Ligands on Hydroxylapatite" BIOCONJUGATE CHEMISTRY, AMERICAN CHEMICAL SOCIETY, WASHINGTON, US, Bd. 8, Nr. 2, 1997, Seiten 103-105, XP002073553 ISSN: 1043-1802**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Verbindungen der Formel I

$$B\text{-}Q\text{-}X_1 \qquad I$$

worin

B ein bioaktives, Integrin-bindendes Molekül
Q fehlt oder ein organisches Spacer-Molekül ausgewählt aus der Gruppe

$[CO\text{-}(CH_2)_x\text{-}NH\text{-}]_m,$ (xii)

$[CO\text{-}CH_2\text{-}(O\text{-}CH_2CH_2)_y\text{-}NH\text{-}]_m,$ (xiii)

$[CO\text{-}(CH_2)_z\text{-}CO\text{-}]$ (xiv)

$[NH\text{-}(CH_2)_z\text{-}NH\text{-}]$ (xv)

$[CO\text{-}CH_2\text{-}(OCH_2CH_2)_y\text{-}O\text{-}CH_2\text{-}CO\text{-}]$ (xvi)

$[NH\text{-}CH_2CH_2\text{-}(OCH_2CH_2)_y\text{-}NH\text{-}]$ (xvii)

sowie deren Kombination
worin
m jeweils unabhängig voneinander 1 bis 20
x 1 bis 12
y 1 bis 50
z 1 bis 12 ist.

$X_1$ ein Ankermolekül, ausgewählt aus der Gruppe

$-Lys\text{-}(CO\text{-}CH_2\text{-}(CH_2)_n\text{-}PO_3H_2)_2$ (i)

$-Lys\text{-}[Lys\text{-}(CO\text{-}CH_2\text{-}(CH_2)_n\text{-}PO_3H_2)_2]_2$ (ii)

oder

$-Lys\text{-}(Lys[\text{-}Lys\text{-}(CO\text{-}CH_2\text{-}(CH_2)_n\text{-}PO_3H_2)_2]_2$ (iii)

bedeutet und n jeweils unabhängig voneinander 0, 1, 2 oder 3 ist, und
wobei eine freie Aminogruppe der Gruppe B mit einer freien Carboxylgruppe des Spacer-Moleküls Q oder des Ankermoleküls $X_1$, bzw. eine freie Aminogruppe des Restes Q mit einer freien Carboxylgruppe des Restes $X_1$ peptidartig miteinander verknüpft ist,
sowie deren Salze.

Ähnliche Verbindungen sind aus DE 19932796, DE 19755800 und DE 19831710 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Vor allem wirken sie als Integrin-Irihibitoren, wobei sie insbesondere die Wechselwirkungen der $\alpha_v\beta_3$- oder $\alpha_v\beta_5$-Integrin-Rezeptoren mit Liganden hemmen, wie z. B. die Bindung von Fibrinogen an den $\beta_3$-Integrinrezeptor. Besondere Wirksamkeit zeigen die Verbindungen im Fall der Integrine $\alpha_v\beta_3$, $\alpha_v\beta_5$, $\alpha_{IIb}\beta_3$ sowie $\alpha_v\beta_1$, $\alpha_v\beta_6$ und $\alpha_v\beta_8$.
Diese Wirkung kann z.B. nach der Methode nachgewiesen werden, die von J.W. Smith et al. in J. Biol. Chem. 265, 12267-12271 (1990) beschrieben wird.
Die Abhängigkeit der Entstehung von Angiogenese von der Wechselwirkung zwischen vaskulären Integrinen und extrazellulären Matrix-proteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.
DE19755801 beschreibt Peptide, die eine für die Zelladhäsion verantwortliche Domäne sowie Ankergruppen enthalten. Die Ankergruppen besitzen u.a. Thiolgruppen, mit denen Sie an das Implantatmaterial binden.

B.C.F Chu et al: "Presentations of Ligands on Hydroxylapatite", publiziert Bioconjugate Chemistry 8, 1997, Seiten 103-105 beschreibt Trimere der 2-Amino-5-Phosphono-Valeriansäure konjugiert an Biotin, die an Hydroxylapatit binden.

Verbindungen der Formel I, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:

Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIlalIlIb-Antagonisten als wirksame Metastase-Hemmer angesehen werden.

Der Phosphonatrest dient dazu, die Peptide ionisch oder adsorptiv an biokompatible Oberflächen von z.B. Implantaten zu binden, die Oxide aufweisen, wie z.B. Metalloberflächen (z.B. Titan bzw. Titanlegierungen wie $TiAl_6V_4$) bzw. Kationen-haltige Oberflächen wie z.B. auf amorphe oder gesinterte Calciumphosphate (z.B. Hydroxylapatit, Knochen, Zähne) oder Calciumphosphatzemente (z.B. Biocement D).

Gegenstand der Erfindung sind daher insbesondere die Verbindungen der Formel I zur ionischen oder adsorptiven Bindung über die funktionelle Gruppe des Restes $X_1$ an biokompatible Oberflächen.

Die erfindungsgemäßen Peptide ermöglichen nun die Biofunktionalisierung von Biomaterialien, insbesondere Implantaten für humane und tierische Organe durch deren Beschichtung, wobei vorwiegend die Adhäsion derjenigen Zellspezies stimuliert wird, die jeweils die Gewebeintegration des entsprechenden Biomaterials vollführen sollen. Mit der Verwendung solcher Beschichtungen ist eine beschleunigte und die verstärkte Integration verschiedener Biomaterialienlimplantate mit verbesserter Langzeitstabilität nach deren Einbringen in den Körper zu erzielen.

Die erfindungsgemäßen Peptide binden selektiv an Integrine. Nach Immobilisierung an biokompatiblen Oberflächen, z.B. Implantaten, stimulieren sie die Adhäsion von Zellen, die Integrine tragen.
Nach Beschichtung der Verbindungen auf den Oberflächen, können selektiv diejenigen Zell-Spezies zur Bindung stimuliert werden, die auch nach Implantation im natürlichen Gewebe die Implantatintegration vollführen sollen. So handelt es sich z.B. bei Osteoblasten, Osteoclasten und Endothelzellen um $\alpha_v$-tragende Zellspezies.

Gegenstand der Erfindung sind daher die Verbindungen der Formel I als Integrininhibitoren zur selektiven Zellanreicherung an Implantaten.

Die Verbindungen der Formel I können nach Verankerung an einer biokompatiblen Oberfläche als Arzneimittelwirkstoffe in der Human- und Veterinärmedizin eingesetzt werden, insbesondere können sie als Integrininhibitoren zur Behandlung von durch Implantate verursachten Erkrankungen, Defekten und Entzündungen wie ungenügender und verzögerter Integration von Biomaterialien und Implantaten, von durch Implantate verursachter Thrombose, von Knochen- und Zahndefekten, sowie von osteolytischen Erkrankungen wie Osteoporose, Thrombose, Herzinfarkt, Arteriosklerose, bei der Wundheilung zur Unterstützung der Heilungsprozesse, sowie zur Beschleunigung und Verstärkung des Integrationsprozesses des Implantats bzw. der biokompatiblen Oberfläche in das Gewebe, eingesetzt werden.

Die Verbindungen der Formel I können als antimikrobiell wirkende Substanzen bei Operationen eingesetzt werden, wo Biomaterialien, Implantate, Katheter oder Herzschrittmacher verwendet werden.
Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

Gegenstand der Erfindung sind somit die Verbindungen der Formel I als integrininhibitoren zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

Gegenstand der Erfindung ist ferner die Verwendung von Verbindungen der Formel I zur Herstellung eines Arzneimittels zur Behandlung von durch Implantate verursachten Erkrankungen, Defekten, Entzündungen und von osteolytischen Erkrankungen wie Osteoporose, Thrombose, Herzinfarkt und Arteriosklerose, sowie zur Beschleunigung und Verstärkung des Integrationsprozesses des Implantats bzw. der biokompatiblen Oberfläche in das Gewebe.

Entsprechende phosphonatankertragende Peptide können ionisch an Träger mit oxidhaltigen Oberflächen,

wie z.B. Implantate, Affinitätschromatographien, oder Mikrotiterplatten oder auch an Kationen-haltige Oberflächen wie z.B. auf amorphe oder gesinterte Calciumphosphate (z.B. Hydroxylapatit, Knochen, Zähne) oder Calciumphosphatzemente (z.B. Biocement D) gebunden werden.

Gegenstand der Erfindung ist auch die Verwendung von Verbindungen der Formel I zur Beschichtung mittels ionischer oder adsorptiver Bindung von Implantaten für humane und tierische Organe.

Die vor- und nachstehend aufgeführten Abkürzungen von Aminosäureresten stehen für die Reste folgender Aminosäuren:

| | |
|---|---|
| Abu | 4-Arninobuttersäure |
| Aha | 6-Aminöhexansäure, 6-Aminocapronsäure |
| Ala | Alanin |
| Asn | Asparagin |
| Asp | Asparaginsäure |
| Arg | Arginin |
| Cys | Cystein |
| Dab | 2,4-Diaminobuttersäure |
| Dap | 2,3-Diaminopropionsäure |
| Gln | Glutamin |
| Glp | Pyroglutaminsäure |
| Glu | Glutaminsäure |
| Gly | Glycin |
| His | Histidin |
| homo-Phe | homo-Phenylalanin |
| Ile | Isoleucin |
| Leu | Leucin |
| Lys | Lysin |
| Met | Methionin |
| Nle | Norleucin |
| Orn | Ornithin |
| Phe | Phenylalanin |
| Phg | Phenylglycin |
| 4-Hal-Phe | 4-Halogen-phenylalanin |
| Pro | Prolin |
| Ser | Serin |
| Thr | Threonin |
| Trp | Tryptophan |
| Tyr | Tyrosin |
| Val | Valin. |

Ferner bedeuten nachstehend:

| | |
|---|---|
| Ac | Acetyl |
| BOC | tert.-Butoxycarbonyl |
| CBZ oder Z | Benzyloxycarbonyl |
| DCCI | Dicyclohexylcarbodümid |
| DMF | Dimethylformamid |
| EDCI | N-Ethyl-N,N'-(dimethylaminopropyl)-carbodiimid |
| Et | Ethyl |
| FCA | Fluoresceincarbonsäure |
| FITC | Fluoresceinisothiocyanat |
| Fmoc | 9-Fluorenylmethoxycarbonyl |
| FTH | Fluoresceinthiohamstoff |
| HOBt | 1-Hydroxybenzotriazol |
| Me | Methyl |
| MBHA | 4-Methyl-benzhydrylamin |
| Mtr | 4-Methoxyt-2,3,6-trimethylphenyl-äulfonyl |
| HATU | O-(7-Azabenzotriazol-1-yl)-N,N,N';N'-Tetramethyluronium-hexafluorophosphat |
| HONSu | N-Hydroxysuccinimid |

| | |
|---|---|
| OtBu | tert.-Butylester |
| Oct | Octanoyl |
| OMe | Methylester |
| OEt | Ethylester |
| POA | Phenoxyacetyl |
| Pbf | Pentamethylbenzofuranyl |
| Pmc | 2,2,5,7,8-Pentamethylchroman-6-sulfonyl |
| Sal | Salicyloyl |
| Su | Succinyl |
| TIPS | Triisopropylsilan |
| TFA | Trifluoressigsäure |

Man kann statt der erfindungsgemäßen Verbindungen auch sogenannte Prodrug-Derivate verwenden, d. h. mit Alkyl- oder Acylgruppen, Zuckern oder Oligopeptiden abgewandelte Verbindungen der Formel 1, die im Organismus rasch zu den wirksamen erfindungsgemäßen Verbindungen gespalten werden.

| | |
|---|---|
| TMSBr | Trimethylsilylbromid |
| Trt | Trityl (Triphenylmethyl). |

Sofern die vorstehend genannten Aminosäuren in mehreren enantiomeren Formen auftreten können, so sind vor- und nachstehend, z. B. als Bestandteil der Verbindungen der Formel 1, alle diese Formen und auch ihre Gemische (z. B. die DL-Formen) eingeschlossen. Ferner können die Aminosäuren, z. B. als Bestandteil von Verbindungen der Formel 1, mit entsprechenden an sich bekannten Schutzgruppen versehen sein.
Vor allem Seitenkettenmodifikationen des Arginins, wie sie z.B. bei den nichtpeptidischen $\alpha_v\beta_3$-Antagonisten vorgenommen wurden (z.B. durch R.Keenan et al., Abstr. Pap. 211th ACS National Meeting (New Orleans, USA) 1996, MEDI 236), können auch bei den Cydopeptiden eingesetzt werden, wie z.B. Benzimidazolderivate anstelle der Guanidingruppe.
Gegenstand der Erfindung ist ferner ein Implantat, geeignet für humane und tierische Organe, bestehend aus einer Trägermatrix und einer diese Matrix umhüllenden Schicht eines bioaktiven, zelladhäsionsvermittelnden Moleküls, wobei die umhüllende Schicht aus einer Verbindung der Formel I gebildet wird, und wobei zwischen Trägermatrix und dieser Verbindung eine ionische oder adsorptive Bindung vorliegt. Vorzugsweise besteht die Trägermatrix und/oder deren Oberfläche aus einem Metall bzw. Metalloxid. Besonders bevorzugt besteht die Trägermatrix und/ oder deren Oberfläche aus einem Knochen- oder Zahnersatzmaterial wie z.B. aus Calciumphosphat-Mischungen.
Gegenstand der Erfindung ist ferner ein Verfahren zur Herstellung von Verbindungen der Formel I nach Anspruch 1 sowie ihrer Salze, dadurch gekennzeichnet, daß man ein bioaktives Molekül B, welches mit Schutzgruppen versehen sein kann, und ein mit Schutzgruppen versehenes Spacer-Anker-Molekül (Q-$X_1$) bzw. Anker-Motekül ($X_1$) peptidisch miteinander verknüpft und anschließend die Schutzgruppen abspaltet, und/oder dass man eine basische oder saure Verbindung der Formel 1 durch Behandeln mit einer Säure oder Base in eines ihrer Salze überführt
Vor- und nachstehend haben die Reste B, Q und $X_1$ die bei der Formel I angegebene Bedeutung, sofern nicht ausdrücklich etwas anderes angegeben ist.
B bedeutet vorzugsweise ein cyclo-(Arg-Gly-Asp-$Z_1$)-,

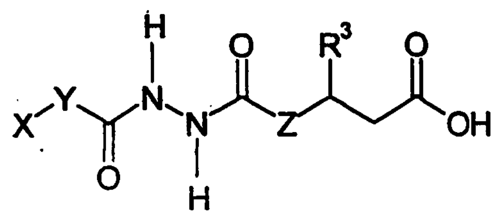
,

Thr-Trp-Tyr-Lys-Ile-Ala-Phe-Gln-Arg-Asn-Arg-Lys-,
Trp-Tyr-Lys-Ile-Ala-Phe-Gln-Arg-Asn-Arg-Lys-,
Tyr-Lys-Ile-Ala-Phe-Gln-Arg-Asn-Arg-Lys-,
Thr-Trp-Tyr-Lys-Ile-Ala-Phe-Gln-Arg-Asn-Arg-,
Thr-Trp-Tyr-Lys-Ile-Ala-Phe-Gln-Arg-Asn- oder ein
Thr-Trp-Tyr-Lys-Ile-Ala-Phe-Gln-Arg-Rest,
wobei $Z_1$ jeweils unabhängig voneinander einen Aminosäurerest oder einen Di- oder Tripeptidrest bedeutet, wobei die

Aminosäuren unabhängig voneinander ausgewählt sind aus der Gruppe bestehend aus Ala, Asn, Asp, Arg, Cys, Gln, Glu, Gly, His, Homo-Phe, Ile, Leu, Lys, Orn, Met, Phe, Phg, Pro, Ser, Thr, Trp, Tyr, Val.

Q fehlt oder bedeutet ein organisches Spacer-Molekül. Bevorzugt ist dies ein $[CO-(CH_2)_x-NH-]_m-$, $[CO-CH_2(-O-CH_2CH_2)_y-NH-]_m-$, $[CO-(CH_2)_z-CO-]-$, $[NH-(CH_2)_z-NH-]-$, $[CO-CH_2(OCH_2CH_2)_y-O-CH_2-CO-]-$ oder ein $[NH-CH_2CH_2-(OCH_2CH_2)_y-NH-]-$ Rest sowie deren Kombinationen, wobei für die Indices m, x, y und z die im Anspruch 3 beanspruchten Wertebereiche gelten. Als besonders vorteilhaft haben sich die vorgenannten Verbindungen erwiesen, die für m Werte zwischen 1 und 8, für x Werte zwischen 1 und 5 und für y und z Werte zwischen 1 und 6 annehmen können.

$X_1$ bedeutet ein Ankermolekül, vorzugsweise aus der Gruppe $-Lys-(CO-CH_2-(CH_2)_n-PO_3H_2)_2$, $-Lys-[Lys-(CO-CH_2-(CH_2)_n-PO_3H_2)_2]_2$ oder-$Lys-(Lys[-Lys-(CO-CH_2-(CH_2)_n-PO_3H_2)_2]_2)_2$, wobei n jeweils unabhängig voneinander 0,1, 2 oder 3 sein kann.

Die in den Bedeutungen für $Z_1$ genannten Aminosäuren und Aminosäurereste können auch derivatisiert sein, wobei die N-Methyl-, N-Ethyl-, N-Propyl-, N-Benzyl- oder $C_\alpha$-Methylderivate bevorzugt sind. Weiter bevorzugt sind Derivate von Asp und Glu, insbesondere die Methyl-, Ethyl, Propyl, Butyl, tert.-Butyl, Neopentyl- oder Benzylester der Seitenkettencarboxy-gruppen, ferner auch Derivate von Arg, das an der $-NH-C(=NH)-NH_2$-Gruppe mit einem Acetyl-, Benzoyl-, Methoxycarbonyl- oder Ethoxycarbonylrest substituiert sein kann.

X bedeutet vorzugsweise $H_2N-C(=NH)-NH-$, Het-NH-, $H_2N-C(=NH)-$, A-C(=NH)-NH oder ein Het-Rest.

Y bedeutet vorzugsweise $-(CH_2)_n-$ oder

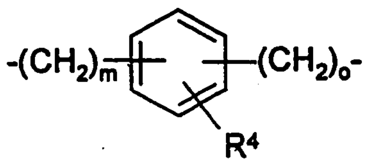

,

$-(CH_2)_s-CH(R^4)-(CH_2)_t-$ oder $-(CH_2)_p-Het^1-(CH_2)_q$-Rest.

Z bedeutet vorzugsweise $N-R^2$ oder $CH-R^2$, wobei $R^2$ vorzugsweise ein H-Atom oder Alkyl-Rest mit 1 bis 4 C-Atomen sein kann.

$R^3$ bedeutet vorzugsweise ein H-Atom, Ar, Het oder A -Rest, wobei A, Ar und Het eine der zuvor oder nachstehend angegebenen Bedeutungen haben.

$R^4$ bedeutet vorzugsweise ein H-Atom, A, Ar, OH, OA, OAr, Arylalkyl, Hal, CN, $NO_2$, $CF_3$ oder $OCF_3$ -Rest. Arylalkyl bedeutet bevorzugt Benzyl, Phenylethyl, Phenylpropyl oder Naphthylmethyl, besonders bevorzugt Benzyl.

A bedeutet vorzugsweise ein COOH-, $NH_2$ - oder Alkyl-Rest mit 1 bis 6 C-Atomen, unsubstituiert oder substituiert mit COOH oder $NH_2$. A bedeutet vorzugsweise Methyl, weiterhin Ethyl, Propyl, n-Butyl, Isobutyl, sek.-Butyl oder tert. Butyl, ferner auch n-Pentyl, 1-,2- oder 3-Methylbutyl, 1,1-, 1,2- oder 2,2-Dimethylpropyl, 1-Ethylpropyl, Hexyl, 1-,2-,3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Besonders bevorzugt für A ist Methyl.

Ar bedeutet vorzugsweise unsubstituiertes oder ein-, zwei- oder dreifach durch A, OH, OA, $CF_3$, $OCF_3$, CN, $NO_2$ oder Hal substituiertes Phenyl, welches durch ein-, zwei- oder dreifach durch A, OH, OA, $NH_2$, $OCF_3$, CN, $NO_2$ oder Hal substituiertes Phenyl in der Art substituiert sein kann, dass ein unsubstituiertes oder substituiertes Biphenyl entsteht.

Ar bedeutet daher bevorzugt Phenyl, o-,m- oder p-Methylphenyl, o-, m-oder p-Ethylphenyl, o-m-oder p-Propylphenyl, o-m- oder p-Isopropylphenyl, o-, m- oder p-tert. Butylphenyl, o-, m- oder p-Hydroxyphenyl-, o-, m- oder p-Methoxyphenyl, o-, m- oder p- Ethoxyphenyl-, o-, m-, p-Trifluormethylphenyl, o-, m-, p-Trifluormethoxyphenyl, o-, m-oder p-Fluorphenyl, o-, m- oder p-Chlorphenyl, o-, m- oder p-Bromphenyl, 0-, m-. p-Nitrophenyl, o-,m- oder p-Aminomethylphenyl.

Het bedeutet einen gesättigten, teilweise oder vollständig ungesättigten mono- oder bicyclischen heterocylcischen Rest mit 5 bis 10 Ringgliedern,

wobei 1 bis 3 N- und/ oder 1S- oder O-Atome vorliegen können und der heterocyclische Rest ein- oder zweifach durch CN, Hal, OH, $NH_2$, COOH, OA, $CF_3$, A, $NO_2$, Ar oder $OCF_3$ substituiert sein kann.

Het ist vorzugsweise ein o-, m-, p-substituiertes Pyridyl, ein 2-, 4-, 5- oder 6-substituiertes Pyrimidyl oder ein 3-, 4-, 5- oder 6-substituiertes Pyridazyl, das bevorzugt unsubstituiert oder substituiert durch eine Methyl-, Ethyl-, Propylgruppe oder eine Methylamino-, Ethylamino- oder Propylaminogruppe ist (bezieht sich auf alle der drei genannten Heteroaromaten), sowie ein 2- substituiertes Benzimidazolyl, das unsubstituiert oder durch eine 3-Methyl-, 3-Ethyl- oder 3-Benzylgruppe substituiert ist, sowie ein 2-substituiertes Dihydroimidazolyl, Tetrahydropyrimidyl oder Tetrahydropyridyl. Beispiele, die in Het bevorzugt enthalten sind:

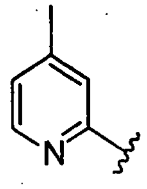 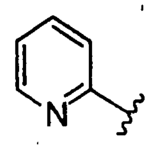 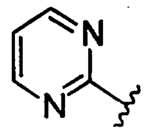 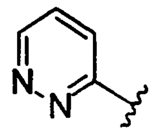

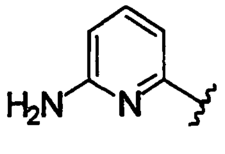 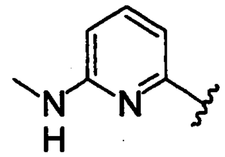 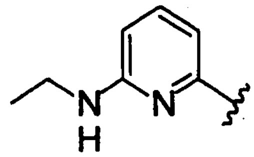 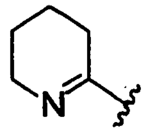

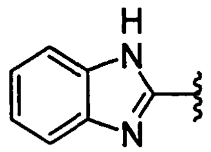 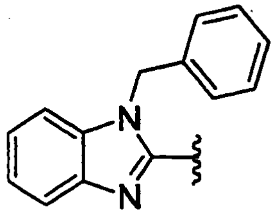 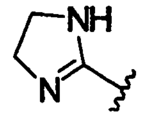 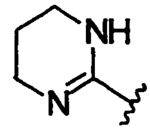

Het[1] bedeutet einen 5- oder 6-gliedrigen aromatischen Heterocyclus mit 1 bis 4 N-, O- und/ oder S-Atomen, der unsubstituiert oder ein- oder zweifach durch F, Cl, Br, A, OA oder $OCF_3$ substituiert sein kann.

Het[1] ist vorzugsweise ein 2,4-, 3,5-, 2,5-disubstituiertes Pyridyl oder ein 2,4-, 2,5-, 2,6-, 4,6-disubstituiertes Pyrimidyl, ein 2,4-, 2,5-disubstituiertes 1,3-Oxazolyl oder 1,3-Thiazolyl.

OA bedeutet vorzugsweise Methoxy, Ethoxy, Propoxy oder Butoxy, ferner auch Pentyloxy oder Hexyloxy.

Hal bedeutet vorzugsweise F, Cl, oder Br, aber auch 1.

Die Indices n, m, o, p, q, s und t haben die im Anspruch 2 angegebene Bedeutung, sofern nicht ausdrücklich etwas anderes angegeben ist.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Besonders bevorzugt sind folgende Verbindungen der Formel I:

a) Cyclo-(Arg-Gly-Asp-D-Phe-Lys($N^{\varepsilon}H$-[CO-$(CH_2)_5$-NH]$_2$-Lys-[Lys-(CO-$CH_2$-$(CH_2)_n$-$PO_3H_2)_2]_2$));
b) Cyclo-(Arg-Gly-Asp-D-Phe-Lys($N^{\varepsilon}H$-[CO-$(CH_2)_5$-NH]$_3$-Lys-[Lys-(CO-$CH_2$-$(CH_2)_n$-$PO_3H_2)_2]_2$));
c) Cyclo-(Arg-Gly-Asp-D-Phe-Lys($N^{\varepsilon}H$-[CO-$CH_2$(-O-$CH_2CH_2)_6$-, NH]$_2$-Lys-[Lys-(CO-$CH_2$-$(CH_2)_n$-$PO_3H_2)_2]_2$));

worin n gleich 1 ist.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Fragmentkupplung bzw. die Kupplung von Ligand an Linker erfolgt in der Regel in einem inerten Lösungsmittel, wobei ein Carbonsäurefragment (Phosphonatlinker z.B. HO-[CO-$(CH_2)_5$-NH]$_2$-Lys-[Lys-(CO-$CH_2$-$CH_2$-$PO_3(C_2H_5)_2)_2]_2$) mit HATU, HOAt und 2,4,6-Collidin in DMF gelöst und mit einem Aminfragment (Cyclopeptid z.B. c[R(Pbf) G(OtBu)fK])) versetzt wird.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran. (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, N-Methylpyrrolidon, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Cyclische Verbindungen können durch Cyclisierung der linearen Verbindungen hergestellt werden, wie z. B. in DE 43 10 643, in Houben-Weyl, l.c., Band 15/II, Seiten 1 bis 806 (1974) oder von S. Zimmer, E. Hoffmann, G. Jung und H. Kessler, Liebigs Ann. Chem. 1993, 497-501, beschrieben.
Die linearen Peptide können z.B. nach R.B. Merrifield, Angew. Chemie 1985, 97, 801-812, synthetisiert werden.

Offenkettige lineare Verbindungen, wie z.B. Verbindungen der Formel I können im übrigen nach üblichen Methoden der Aminosäure- und Peptidsynthese hergestellt werden, z. B. auch nach der Festphasensynthese nach Merrifield (s. auch z.B. B. F. Gysin und R. B. Merrifield, J. Am. Chem. Soc. 94, 3102 ff. (1972)).

Die Verbindungen der Formel I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel 1 entsprechen, aber anstelle einer $NH_2$-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel 1 entsprechen, aber anstelle einer Hydroxyphenylgruppe eine R"O-phenylgruppe, enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Iodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OtBu)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Über schuß ohne Zusatz eines weiteren Lösungsmittels verwendet, Perchlorsäure in Form eines Gemisches aus Essigsäure und 70 %iger Perchlor säure im Verhältnis 9:1. Die Reaktionstemperaturen für die Spaltung liegen zweckmäßig zwischen etwa 0 und etwa 50°,

vorzugsweise arbeitet man zwischen 15 und 30° (Raumtemperatur).

Die Gruppen BOC, OtBu, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird zum Schutz der Aminosäuren Histidin, Asparagin, Glutamin und Cystein eingesetzt. Die Abspaltung erfolgt, je nach gewünschtem Endprodukt, mit TFA /10% Thiophenol, wobei die Tritylgruppe von allen genannten Aminosäuren abgespalten wird, bei Einsatz von TFA / Anisol, TFA / Thioanisol oder TFA/TIPS/$H_2O$ wird nur die Tritylgruppe von His, Asn und Gln abgespalten, wogegen sie an der Cys-Seitenkette verbleibt.
Die Pbf (Pentamethylbenzofuranyl)-gruppe wird zum Schutz von Arg eingesetzt. Die Abspaltung erfolgt z.B. mit TFA in Dichlormethan.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° C und Drucken zwischen etwa 1 und 200 bar, bevorzugt bei 10-30° C und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 10-30° C.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfäminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyloder Diisopropyl-ammoniumsalze, Monoethanol-, Diethanol- oder Diisopropanolammoniumsalze, Cyclohexyl-, Dicyclohexylammoniumsalze, Dibenzylethylendiammoniumsalze, weiterhin z. B. Salze mit Arginin oder Lysin.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
RZ = Retentionszeit (Minuten) bei HPLC in den folgenden Systemen:
[A]

| | |
|---|---|
| Säule: | YMC ODS A RP 5$C_{18}$, 250 x 4,6 mm |
| Eluent A: | 0,1 % TFA in Wasser |
| Eluent B: | 0,1 % TFA in Acetonitril |
| Fluß: | 1 ml/min |
| Gradient: | 0 - 50 % B / 30 min. |

[B]
wie [A];

Gradient: 5 - 50 % B / 30 min.

[C]
wie [A];

| | |
|---|---|
| Gradient: | 10 - 50 % B / 30 min. |
| Massenspektrometrie (MS): | EI (Elektronenstoß-Ionisation) $M^+$ |
| | FAB (Fast Atom Bombardment) $(M+H)^+$ |
| | ESI (Elektrospray-Ionisation) $(M+H)^+$ |

DMPP-Harz steht für 4-(2',4'-Dihiethoxyphenyl-hydroxymethyl)phenoxy-Harz, welches z.B. die Synthese von seitenkettengeschützten Peptiden erlaubt, TCP-Harz bedeutet Tritylchlorid-Polystyrol-Harz.

Die nachfolgenden Beispiele beschreiben zum einen die Fragmentkupplung sowie die Spaltung von Phosphonestem und zum anderen die Synthese ausgewählter Cyclopeptid-Derivate mit Phosphonatlinker. Anhand der Beispiele 7 bis 9 wird das Verfahren zur Beschichtung der verschiedenen Formkörper aus Metall oder Knochenersatzmaterialien näher erläutert.

Beispiel 1: Fragmentkupplung in Lösung

0.2 mmol Carbonsäurefragment (Phosphonatlinker, z. B. HO-[CO-$(CH_2)_5$-NH]$_2$-Lys-[Lys-(CO-$CH_2$-$CH_2$-$PO_3$$(C_2H_5)_2)_2]_2$), 0.98 eq HATU, 1:1eq HOAt und 10 eq 2,4,6-Collidin werden in 2 mL DMF gelöst. Nach 1.5 h wird 1 eq Aminfragment (Cyclopeptid, z. B. c[R(Pbf)G(OtBu)fK]) zugegeben. Man läßt 24 h bei Raumtemperatur rühren und reinigt das Produkt mittels präparativer HPLC.

Beispiel 2: Spaltung von Phosphonestem bei den Phosphonatlinkem

Das Peptid mit Phosphonestergruppen wird in einer Mischung von abs. $CHCl_3$ und TMSBr 10 : 1 gelöst bzw. ggf. im Ultraschallbad suspendiert. Nach 3 Tagen Rühren und ggf. gelegentlichem Aufschlämmen des Niederschlags im Ultraschallbad wird das Lösungsmittel abdestilliert. Der Rückstand wird aus $H_2O$ lyophilisiert.

Die Abspaltung der sauren Seitenkettenschutzgruppen erfolgt nach den gängigen Arbeitstechniken.

Beispiel 3: Synthese der Phosphonatlinker

Die Phosphonatlinker wurden in einer Festphasenpeptidsynthese nach der Fmoc-Strategie synthetisiert (siehe G.B. Fields, R. L. Nobie, Int. J. Pept. Protein Res. 1990, 35, 161-214).
Als letzter Baustein wurde 3-Diethylphosphonopropionsäure gekuppelt.

Synthese von 3-Diethylphosphonopropionsäure:

3-Brompropionsäurebenzylester

58.3 mmol 3-Brompropionsäurechlorid (10.0 g) und 1 eq Benzylalkohol (6.3 g) werden in 100 mL trockenem DCM unter Rühren in einem 250 mL Rundkolben mit Calciumchloridrohr gelöst. Nach 2 Tagen gibt man zum Ansatz 200 mL $CHCl_3$ und extrahiert die organische Phase 2 mal mit gesättigter $NaHCO_3$-Lösung. Nach Trocknen über $MgSO_4$ destilliert man das Lösungsmittel ab, man erhält das Produkt als farblose Flüssigkeit.
Ausbeute: 13.8 g (56.8 mmol, 97%); $R_f$ = 0.75 (H:EE 1:1).

NMR ($CDCl_3$):
$^1H$ (250 MHz): $\delta$ = 7.35 (s, 5H; H ar), 5.16 (s, 2H; $CH_2$-OCO), 3.58 (t, $^3J$(H,H) = 7 Hz, 2H; $CH_2Br$), 2.95 (t, $^3J$(H,H) = 7 Hz, 2H; $CH_2CO$). GC-MS:$m^+$=242.0.

3-Diethylphosphonopropionsäurebenzylester

56.8 mmol 3-Brompropionsäurebenzylester (13.8 g) und 1.7 eq Triethylphosphit (16.0 g) werden unter Rühren in einer mit Gasballon als Druckausgleich abgedichteten Destillationsapparatur auf 140°C erhitzt. Das im Laufe der Reaktion gebildete Bromethan wird kontinuierlich abdestilliert und im auf 0 °C gekühlten Vorlagekolben aufgefangen. Nach 4 h wird der verbleibende ölige Rückstand im Hochvakuum fraktionierend über eine Vigreux-Kolonne destilliert, das Produkt ist ein farbloses Öl. Ausbeute: 12.1 g (40.3 mmol, 71%), leicht verunreinigt, $R_f$ = 0.33 (A:H 2:3).
Für höhere Reinheit ist das Produkt ggf. flash-chromatographisch zu reinigen (Eluent A:H 2:3).

NMR ($CDCl_3$):
$^1H$ (250 MHz): $\delta$= 7.31 (s, 5H; H ar), 5.10 (s, 2H, $CH_2$-OCO), 4.05 (m, 4H; $CH_2OP$), 2.61 (m, 2H; $CH_2CO$), 2.04 (m, 2H; $CH_2P$), 1.26 (t, $^3J$(H,H) = 7 Hz, 6H; $CH_3$).

$^{31}P$ (101.256 MHz): δ= 28.5 (s).
GC-MS: $m^+$= 300.0.

3-Diethylphosphonopropionsäure

40.3 mmol 3-Diethylphosphonopropionsäurebenzylester (12.1 g) werden in 100 mL Ethanol gelöst und mit 2 g Katalysator (5% Pd / C) versetzt. Nach 4 h Rühren unter $H_2$-Atmosphäre wird die Aktivkohle abfiltriert, das Lösungsmittel wird abdestilliert. Das Produkt fällt als farbloses Öl an, das bei Raumtemperatur langsam zu einem farblosen Feststoff erstarrt. Das Produkt ist leicht verunreinigt mit 3-Diethylphosphonopropionsäureethylester, der gegebenenfalls durch mehrfaches Ausschütteln zwischen Wasser und Hexan in die organische Phase überführt und abgetrennt werden kann. Ausbeute: 8.2 g (39.0 mmol, 97%).
NMR ($CDCl_3$):
$^1H$ (250 MHz): δ = 10.60 (bs, 1H; COOH), 4.07 (m, 4H; $CH_2O$), 2.59 (m, 2H; $CH_2CO$), 2.06 (m , 2H; $CH_2P$), 1.29 (t, $^3J$ (H,H) = 7 Hz, 6H; $CH_3$).
$^{13}C$ (62.896 MHz): δ= 174.5 (d, *J*(C,P) = 18.5 Hz ; COOH), 62.1 (d, *J*(C,P) = 6.6 Hz ; $CH_2$-O), 27.1 (d, *J*(C,P) = 3.8 Hz; $CH_2$-COOH), 20.7 (d, *J*(C,P) = 144.9 Hz; $CH_2$-P), 16.2 (d, *J*(C,P) = 6.1 Hz; $CH_3$).
$^{31}P$ (101.256 MHz): δ= 29.5 (s).

Analytische Daten der Cyclopeptide mit Phosphonatlinker:

Beispiel 4: (2 Aminohexansäuren im Spacer)

Cyclo-(Arg-Gly-Asp-DPhe-Lys($N^8H$-[CO-$(CH_2)_5$-NH]$_2$-Lys-[Lys-(CO-$CH_2$-$CH_2$-$PO_3H_2$)$_2$]$_2$))

MS (ESI): *m/z* (%): 1756.9 (100) [m - $H^+$], 1778.8 (48) [m + $Na^+$ - $2H^+$], 1794.9 (18) [m + $K^+$ - $2H^+$].
NMR ([$D_6$]DMSO):
$^{31}P$ (101.256 MHz): δ= 29.63 (s, 1P), 29.59 (s, 1P), 29.57 (s, 1P), 29.38 (s, 1P).

Beispiel 5: (3 Aminohexansäuren im Spacer)

Cyclo-(Arg-Gly-Asp-DPhe-Lys($N^8H$-[CO-$(CH_2)_5$-NH]$_3$ -Lys-[Lys-(CO-$CH_2$-$CH_2$-$PO_3H_2$)$_2$]$_2$))
MS (ESI): *m/z* (%): 934.9 (100) [m - $2H^+$], 1870.0 (27) [m - $H^+$].
NMR ([$D_6$]DMSO):
$^{31}P$ (101.256 MHz): δ= 29.57 (s, 1P). 29.51 (s, 2P), 29.33 (s, 1 P).

Beispiel 6: (2 Heptaethylenglycolaminocarbonsäuren im Spacer)

Cyclo-(Arg-Gly-Asp-DPhe-Lys($N^{\varepsilon}H$-[CO-$CH_2$(-O-$CH_2CH_2$)$_6$-NH]$_2$-Lys-[Lys-(CO-$CH_2$-$CH_2$-$PO_3H_2$)$_2$]$_2$))
MS (ESI): *mlz* (%): 1086.4 (100) [m - $2H^+$], 1097.7 (73) [m + $Na^+$- $3H^+$]. NMR ([$D_6$]DMSO):
$^{31}P$ (101.256 MHz): δ= 29.66 (s, 1P). 29.61 (s. 1 P), 29.59 (s, 1P). 29.45 (s, 1P).
Zudem wurden alle vorgenannten Peptide durch $^1H$ NMR-Spektroskopie (250 MHz) charakterisiert, die erwarteten Spektren wurden erhalten.

Beispiel 7:

Ti- bzw. $TiAt_6V_4$-Formkörper mit einem Durchmesser von 10 mm und einer Höhe von 1-2 mm werden 15 min. in destilliertem Wasser bei 60° C im Ultraschallbad vorgereinigt, anschließend 30 min. mit Aceton und danach zweimal mit destilliertem Wasser gewaschen und acht Stunden im Trockenschrank getrocknet.
Die Formkörper werden in 48-wells (Costar, "non-tissue culture treated" Art. Nr. 3574) transferiert. Zur Anbindung der bioaktiven, zelladhäsionsvermittelnden Moleküle B (wobei B ein Cyclopeptid, Peptidmimetikum oder lineares Peptid gemäß Anspruch 2 sein kann) an die vorbereiteten Formkörper werden Moleküle B -enthaltende Stammlösungen ("B-Lösungen") in einer Endkonzentration von 1 mM in einem der wäßrigen Puffer Tris-HCl(10 mM, pH 8.7), Tris-$HClO_4$ (10 mM, pH 8.7) oder PBS, pH 7.4 hergestellt. Anschließend werden durch Verdünnung mit PBS, pH 7.4, Konzentrationsreihen mit den "B-Lösungen"-Endkonzentrationen von jeweils 1 nM, 10 nM, 100 nM, 1 μM, 10 μM und 100 μM hergestellt.
Die Formkörper werden mit je 250 μl der jeweiligen B-Lösung überschichtet und anschließend für 18-24 Stunden bei Raumtemperatur inkubiert. Zur Entfernung ungebundener B-Moleküle wurden die Proben dreimal mit PBS, pH 7.4 gewaschen und über Nacht in PBS, pH 7.4 bei 4 °C gelagert.

Durch Zugabe von je 250 µl/ Formkörper einer 5%igen BSA (Bovine Serum Albumine)-Lösung, pH 7.4, anschließender Inkubation für 2 Stunden bei Raumtemperatur und einmaligem Waschen mit PBS, pH 7.4 werden unspezifische Zellbindungsstellen blockiert.
Als Negativkontrollen fungieren Ti- bzw. $TiAl_6V_4$-Formkörper, die anstelle von B-Lösungen mit entsprechenden Pufferlösungen (Tris-HCl 10 mM, pH 8.7; Tris-$HClO_4$ 10 mM, pH 8.7; PBS, pH 7.4) behandelt werden.
Der Grad der erhaltenen Beschichtungen auf den Formkörpern wird analytisch bewertet und die biologische Wirksamkeit mittels eines Zelladhäsionstests in vitro bestimmt.

Beispiel 8:

Elfenbeinformkörpef als Modell für ein natürliches Knochenersatzmaterial mit einem Durchmesser von 10 mm und einer Höhe von 100 µm werden durch Fräsen von Zylindern mit 10 mm Durchmesser und anschließendes. Sägen in 100 µm Dicke in einer Buehler ISOMET "low speed saw" hergestellt. Anschließend werden die Proben 10 min. in dest. Wasser bei 60 °C im Ultraschallbad gereinigt und danach zweimal mit dest. Wasser gewaschen und 8 Stunden im Trockenschrank getrocknet.
Zur Beschichtung der Formkörper mit B-Lösungen wird wie unter Beispiel 7 beschrieben verfahren.
Der Grad der erhaltenen Beschichtung auf den Formkörper wird analytisch bewertet und die biologische Wirksamkeit mittels eines Zelladhäsionstests in vitro bestimmt

Beispiel 9:

Formkörper des kommerziell erhältlichen Knochenersatzmaterials Endobon® (Fa. Biomet Merck, Deutschland) mit einem Durchmesser von 10 mm und einer Höhe von 100 µm werden durch Fräsen von Zylindern mit 10 mm Durchmesser und anschließendes Sägen in 100 µm Dicke in einer Buehler ISOMET "low speed saw" hergestellt. Anschließend werden die Proben 10 min in destilliertem Wasser bei 60 °C im Ultraschallbad gereinigt und danach zweimal mit destilliertem Wasser gewaschen und 8 Stunden im Trockenschrank getrocknet.
Zur Beschichtung der Formkörper mit der "B-Lösung" wird wie unter Beispiel 7 beschrieben verfahren.
Der Grad der erhaltenen Beschichtung auf den Formkörpern wird analytisch bewertet und die biologische Wirksamkeit mittels eines Zelladhäsionstests in vitro bestimmt.

Beispiel für Zelladhäsionstest:

Es wurde die Adhäsion von Maus-MC3T3 H1-Osteoblastenkultufen in vitro an RGD-Peptid-beschichteten Materialoberflächen untersucht. Dabei wurden 50.000 Zellen/$cm^2$ angesät und nach einstündiger Inkubation in serumfreiem Medium bei 37° C / 95 % Luftfeuchte der Anteil adhärierter Zellen bestimmt.

**Zellanhaftungsrate [%] = adherierte Zellen / angesäte Zellen x 100**

Peptid: Zellanhaftungsrate [%]

Cyclo-(Arg-Gly-Asp-DPhe-Lys($N^8$H-[CO-$(CH_2)_5$-NH$]_2$ -Lys-[Lys-(CO-$CH_2$-$CH_2$-$PO_3H_2)_2]_2$)): 75

Cyclo-(Arg-Gly-Asp-DPhe-Lys($N^{\varepsilon}$H-[CO-$(CH_2)_5$-NH$]_3$-Lys-[Lys-(CO-$CH_2$-$CH_2$-$PO_3H_2)_2]_2$)): 62

## Patentansprüche

**1.** Verbindung der Formel 1

B-Q-X1

worin

B ein bioaktives, Integrin-bindendes Molekül
Q fehlt oder ein organisches Spacer-Molekül ausgewählt aus der Gruppe,

$[CO\text{-}(CH_2)_x\text{-}NH\text{-}]_m.$ (xii)

$[CO\text{-}CH_2\text{-}(O\text{-}CH_2CH_2)_y\text{-}NH\text{-}]_m,$ (xiii)

$[CO\text{-}(CH_2)_2\text{-}CO\text{-}]$ (xiv)

$[NH\text{-}(CH_2)_z\text{-}NH\text{-}]$ (xv)

$[CO\text{-}CH_2\text{-}(OCH_2CH_2)_y\text{-}O\text{-}CH_2\text{-}CO\text{-}]$ (xvi)

$[NH\text{-}CH_2CH_2\text{-}(OCH_2CH_2)_y\text{-}NH\text{-}]$ (xvii)

sowie deren Kombination
worin

m jeweils unabhängig voneinander 1 bis 20
x 1 bis 12
y 1 bis 50
z 1 bis 12 ist,

$X_1$ ein Ankermolekül, ausgewählt aus der Gruppe

$-Lys-(CO-CH_2-(CH_2)_n-PO_3H_2)_2$ (i)

$-Lys-[Lys-(CO-CH_2-(CH_2)_n-PO_3H_2)_2]_2$ (ii)

oder

$-Lys-(Lys[-Lys-(CO-CH_2-(CH_2)_n-PO_3H_2)_2]_2$ (iii)

bedeutet und n jeweils unabhängig voneinander 0, 1, 2 oder 3 ist, und wobei eine freie Aminogruppe der Gruppe B mit einer freien Carboxylgruppedes Spacer-Moleküls Q oder des Ankermoleküls $X_1$, bzw. eine freie Aminogruppe des Restes Q mit einer freien Carboxylgruppe des Restes $X_1$ peptidartig miteinander verknüpft ist, sowie deren Salze.

**2.** Verbindung nach Anspruch 1, worin die Gruppe B ausgewählt ist aus der Gruppe

cyclo-(Arg-Gly-Asp-$Z_1$) (iv)

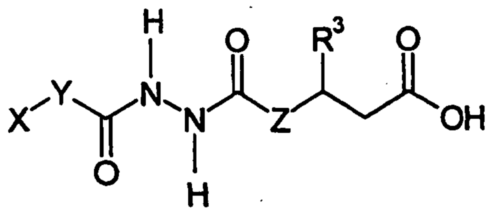

(v)

und

Thr-Trp-Tyr-Lys-Ile-Ala-Phe-Gln Arg-Asn-Arg-Lys (vi)

Trp-Tyr-Lys-lie-Ala-Phe-Gln-Arg-Asn-Arg-Lys (vii)

Tyr-Lys-Ile-Ala-Phe-Gln-Arg-Asn-Arg-Lys (viii)

Thr-Trp-Tyr-Lys-lie-Ala-Phe-Gln-Arg-Asn-Arg (ix)

Thr-Trp-Tyr Lys-Ile-Ala-Phe-Gln-Arg-Asn (x)

Thr-Trp-Tyr-Lys-Ile-Ala-Phe-Gin-Arg (xi)

worin

$Z_1$ jeweils unabhängig voneinander einen Aminosäurerest oder einen Di- oder Tripeptidrest, wobei die Aminosäuren unabhängig voneinander ausgewählt sind aus einer Gruppe bestehend aus Ala, Asn, Asp, Arg, Cys, Gln, Glu, Gly, His, Homo-Phe, Ile, Leu, Lys, Met, Orn, Phe, Phg, Pro, Ser, Thr, Trp, Tyr, Val,

worin für (v)

X $H_2N$-C(=NH)-NH, Het-NH-, $H_2N$-C(=NH)-, A-C(=NH)-NH oder Het-
Y $-(CH_2)_n-$ oder

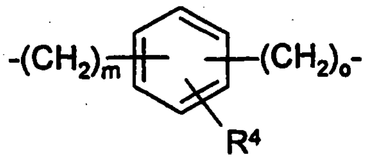 ,

$-(CH_2)_s-CH(R^4)-(CH_2)_r$ oder $-(CH_2)_p-Het^1-(CH_2)_q-$,
Z N-$R^2$ oder CH-$R^2$
$R^2$ H oder Alkyl mit 1 bis 4 C-Atomen
$R^3$ H, Ar, Het oder A
$R^4$ H, A, Ar, OH,OA, OAr, Arylalkyl, Hal ,CN, $NO_2$, $CF_3$ oder $OCF_3$,
A COOH, $NH_2$ oder Alkyl mit 1-6 C-Atomen, unsubstituiert oder substituiert mit COOH oder $NH_2$
Ar unsubstituiertes oder ein-, zwei-oder dreifach durch A, OH, OA, $CF_3$, $OCF_3$, CN, $NO_2$ oder Hal substituiertes Phenyl, welches durch ein ein-, zwei oder dreifach durch A, OH, OA, $NH_2$, $OCF_3$, CN, $NO_2$ oder Hal substituiertes Phenyl in der Art substituiert sein kann, dass ein unsubstituiertes oder substituiertes Biphenyl entsteht,
Hal F, Cl, Br oder I,
Het einen gesättigten, teilweise oder vollständig ungesättigten mono- oder bicyclischen heterocyclischen Rest mit 5 bis 10 Ringgliedern, wobei 1 bis 3 N- und/oder 1 S- oder O-Atome vorliegen können und der heterocyclische Rest ein- oder zweifach durch CN, Hal, OH , $NH_2$, COOH, OA, $CF_3$, A, $NO_2$, Ar oder $OCF_3$ substituiert sein kann,
$Het^1$ einen 5- oder 6-gliedrigen aromatischen Heterocyclus mit 1 bis 4 N- und / oder S-Atomen, der unsubstituiert oder ein- oder zweifach durch F, Cl, Br, A, OA oder $OCF_3$ substituiert sein kann,
n 4, 5 oder 6,
m, o,p, q 0, 1 oder 2
s, t 0, 1, 2, 3, 4 oder 5

**3.** Verbindung nach Anspruch 1 oder 2, worin Q ausgewählt ist aus der Gruppe

$[CO-(CH_2)_x-NH-]_m$, (xviii)

$[CO-CH_2(-O-CH_2CH_2)_y-NH-]_m$ (xix)

$[CO-(CH_2)_z-CO-]$ (xx)

$[NH-(CH_2)_z-NH-]$ (xxi)

$[CO-CH_2-(OCH_2CH_2)_y-O-CH_2-CO-]$ (xxii)

$[NH-CH_2CH_2-(OCH_2CH_2)_y-NH-]$ (xxiii)

sowie deren Kombination
worin

m jeweils unabhängig voneinander 1 bis 8
x 1- 5
y 1- 6 und
z 1- 6 ist.

**4.** Verbindungen der Formel 1 gemäß Anspruch 1

a) Cyclo-(Arg-Gly-Asp-DPhe-Lys($N^{\varepsilon}H$-$[CO-(CH_2)_5-NH]_2$-Lys-$[Lys-(CO-CH_2-(CH_2)_n-PO_3H_2)_2]_2$));
b) Cyclo-(Arg-Gly-Asp-DPhe-Lys($N^{\varepsilon}H$-$[CO-(CH_2)_5-NH]_3$-Lys-$[Lys-(CO-CH_2-(CH_2)_n-PO_3H_2)_2]_2$));
c) Cyclo-(Arg-Gly-Asp-DPhe-Lys($N^{\varepsilon}H$-$[CO-CH_2(-O-CH_2CH_2)_6-NH]_2$- Lys-$[Lys-(CO-CH_2-(CH_2)_n-PO_3H_2)_2]_2$));

worin n gleich 1 ist.

5. Verbindung nach einem der Ansprüche 1 bis 4 zur Verwendung als Arzneimittel zur Behandlung von Metastasen durch Inhibierung der Angiogenese und der Ausbreitung von Tumorzellen, zur antimikrobiellen und antiseptischen Behandlung von Entzündungen und Defekten hervorgerufen durch den Einbau von Implantaten und von osteolytischen Erkrankungen wie Osteoporose, Thrombose. Herzinfarkt und Arteriosklerose, sowie zur Beschleunigung und Verstärkung des Integrationsprozesses von Implantaten bzw. biokompatiblen Oberfläche in das Gewebe durch selektive Zellanreicherung an Implanaten.

6. implantat, geeignet für humane und tierische Organe, bestehend aus einer Trägermatrix und einer diese Matrix umhüllenden Schicht eines bioaktiven, itelladhäsionsverrnftteinderi Moleküls, **dadurch gekennzeichnet; daß** die umhüllende Schicht aus einer Verbindung gemäß der Ansprüche 1 bis 5 gebildet wird, wobei zwischen Trägennatrix und dieser Verbindung eine ionische oder adsorptive Bindung vorliegt.

7. Implantat nach Anspruch 6, **dadurch gekennzeichnet, dass** die Trägermatrix und/oder deren Oberfläche ein Metall oder ein Metalloxid ist.

8. Implantat nach Anspruch 6, **dadurch gekennzeichnet, dass** die Trägermatrix und/oder deren Oberfläche ein Knochen- oder Zahnersatzmaterial ist.

9. Implantat nach Anspruch 8, **dadurch gekennzeichnet, dass** das Knochen- oder Zahnersatzmaterial aus Calciumphosphat-Mischungen besteht.

10. Verfahren zur Herstellung einer Verbindung nach Anspruch 1 sowie ihrer Salze, **dadurch gekennzeichnet, dass** man ein bioaktives Molekül B, welches mit Schutzgruppen versehen sein kann, und ein mit Schutzgruppen versehenes Spacer-Anker-Molekot (Q-$X_1$) bzw. Anker-Molekül ($X_1$) peptidisch miteinander verknüpft und anschließend die Schutzgruppen abspaltet, und/oder dass man eine basische oder saure Verbindung der Formel 1 durch Behandeln mit einer Säure oder Base in eines ihrer Salze überführt.

11. Verwendung einer Verbindung nach einem der Ansprüche 1 bis 4 zur Herstellung eines Arzneimittels zur Behandlung von Metastasen durch Inhibierung der Angiogenese und der Ausbreitung von Tumorzellen, zur antimikrobiellen und antiseptischen Behandlung von Entzündungen und Defekten hervorgerufen durch den Einbau von Implantaten und von osteolytischen Erkrankungen wie Osteoporose, Thrombose, Herzinfarkt und Arteriosklerose, sowie zur Beschleunigung und Verstärkung des Integrationsprozesses von Implantaten bzw. biokompatiblen Oberfläche in das Gewebe durch selektive Zellanreicherung an Implanaten.

12. Verwendung einer Verbindung nach einem der Anprüche 1 bis 5 zur Beschichtung mittels ionischer oder adsorptiver Bindung von Implantaten für humane und tierische Organe.

## Claims

1. Compound of the formula 1

B-Q-X1 1

in which

B is a bioactive, integrin-binding molecule,
Q is absent or is an organic spacer molecule selected from the group

$[CO-(CH_2)_x-NH-]_m$, (xii)

$[CO-CH_2-(O-CH_2CH_2)_y-NH-]_m$, (xiii)

$[CO-(CH_2)_z-CO-]$ (xiv)

$[NH-(CH_2)_z-NH-]$ (xv)

$[CO-CH_2-(OCH_2CH_2)_y-O-CH_2-CO-]$ (xvi)

$[NH-CH_2CH_2-(OCH_2CH_2)_y-NH-]$ (xvii)

and their combination

in which

m in each case independently of one another is 1 to 20,
x is 1 to 12,
y is 1 to 50
z is 1 to 12,
$X_1$ is an anchor molecule, selected from the group

$-Lys-(CO-CH_2-(CH_2)_n-PO_3H_2)_2$ (i)

$-Lys-[Lys-(CO-CH_2-(CH_2)_n-PO_3H_2)_2]_2$ (ii)

or

$-Lys-(Lys[-Lys-(CO-CH_2-(CH_2)_n-PO_3H_2)_2]_2$ (iii),

and n, in each case independently of one another, is 0, 1, 2 or 3, and where a free amino group of the group B and a free carboxyl group of the spacer molecule Q or of the anchor molecule $X_1$, or a free amino group of the radical Q and a free carboxyl group of the radical $X_1$ are linked to one another like peptides, and its salts.

**2.** Compound according to Claim 1, in which group B is selected from the group

cyclo-(Arg-Gly-Asp-$Z_1$) (iv)

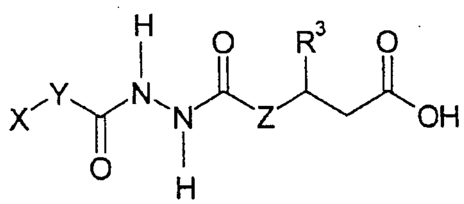

(v)

and

Thr-Trp-Tyr-Lys-Ile-Ala-Phe-Gln-Arg-Asn-Arg-Lys (vi)

Trp-Tyr-Lys-Ile-Ala-Phe-Gln-Arg-Asn-Arg-Lys (vii)

Tyr-Lys-Ile-Ala-Phe-Gln-Arg-Asn-Arg-Lys (viii)

Thr-Trp-Tyr-Lys-Ile-Ala-Phe-Gln-Arg-Asn-Arg (ix)

Thr-Trp-Tyr-Lys-Ile-Ala-Phe-Gln-Arg-Asn (x)

Thr-Trp-Tyr-Lys-Ile-Ala-Phe-Gln-Arg (xi)

where

$Z_1$ is, in each case independently of one another, an amino acid residue or a di- or tripeptide residue, where the amino acids are selected, independently of one another, from a group consisting of Ala, Asn, Asp, Arg, Cys, Gln, Glu, Gly, His, Homo-Phe, Ile, Leu, Lys, Met, Orn, Phe, Phg, Pro, Ser, Thr, Trp, Tyr, Val,

where for (v)

X is $H_2N$-C (=NH)-NH, Het-NH- , $H_2N$-C (=NH)-, A-C(=NH)-NH or Het-,
Y is - $(CH_2)_n$- or

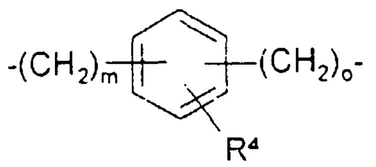

,

$-(CH_2)_s$-CH($R^4$)$-(CH_2)_t$- or $-(CH_2)_p$-$Het^1$$-(CH_2)_q$-,
Z is N-$R^2$ or CH-$R^2$,
$R^2$ is H or alkyl having 1 to 4 C atoms,
$R^3$ is H, Ar, Het or A,
$R^4$ is H, A, Ar, OH, OA, OAr, arylalkyl, Hal, CN, $NO_2$, $CF_3$ or $OCF_3$,
A is COOH, $NH_2$ or alkyl having 1-6 C atoms, unsubstituted or substituted by COOH or $NH_2$,
Ar is phenyl which is unsubstituted or mono-, di- or trisubstituted by A, OH, OA, $CF_3$, $OCF_3$, CN, $NO_2$ or Hal, which can be substituted by a phenyl which is mono-, di- or trisubstituted by A, OH, OA, $NH_2$, $OCF_3$, CN, $NO_2$ or Hal in such a way that an unsubstituted or substituted biphenyl results,
Hal is F, Cl, Br or I,
Het is a saturated, partly or completely unsaturated mono- or bicyclic heterocyclic radical having 5 to 10 ring members, where 1 to 3 N and/or 1 S or O atom(s) can be present and the heterocyclic radical can be mono- or disubstituted by CN, Hal, OH, $NH_2$, COOH, OA, $CF_3$, A, $NO_2$, Ar or $OCF_3$,
$Het^1$ is a 5- or 6-membered aromatic heterocycle having 1 to 4 N and/or S atoms, which can be unsubstituted or mono- or disubstituted by F, Cl, Br, A, OA or $OCF_3$,
n is 4, 5 or 6,
m, o, p, q are 0, 1 or 2,
s, t are 0, 1, 2, 3, 4 or 5.

**3.** Compound according to Claim 1 or 2, in which Q is selected from the group

$[CO-(CH_2)_x-NH-]_m$, (xviii)

$[CO-CH_2(-O-CH_2CH_2)_y-NH-]_m$ (xix)

$[CO-(CH_2)_2-CO-]$ (xx)

$[NH-(CH_2)_z-NH-]$ (xxi)

$[CO-CH_2-(OCH_2CH_2)_y-O-CH_2-CO-]$ (xxii)

$[NH-CH_2CH_2-(OCH_2CH_2)_y-NH-]$ (xxiii)

and their combination
in which

m in each case independently of one another is 1 to 8,
x is 1-5,
y is 1-6 and
z is 1-6.

**4.** Compounds of the formula 1 according to Claim 1

a) Cyclo-(Arg-Gly-Asp-DPhe-Lys($N^{\varepsilon}$H-$[CO-(CH_2)_5-NH]_2$-Lys-[Lys-$(CO-CH_2-(CH_2)_n-PO_3H_2)_2]_2$)):
b) Cyclo-(Arg-Gly-Asp-DPhe-Lys($N^{\varepsilon}$H-$[CO-(CH_2)_5-NH]_3$-Lys-[Lys-$(CO-CH_2-(CH_2)_n-PO_3H_2)_2]_2$));
c) Cyclo-(Arg-Gly-Asp-DPhe-Lys($N^{\varepsilon}$H-$[CO-CH_2(-O-CH_2CH_2)_6-NH]_2$-Lys-[Lys-$(CO-CH_2-(CH_2)_n-PO_3H_2)_2]_2$));

where n is 1.

5. Compound according to one of Claims 1 to 4 for use as a medicament for the treatment of metastases by inhibiting angiogenesis and the spread of tumour cells, for the antimicrobial and antiseptic treatment of inflammations and defects caused by the incorporation of implants and of osteolytic disorders such as osteoporosis, thrombosis, cardiac infarct and arteriosclerosis, and also for the acceleration and strengthening of the integration process of implants or biocompatible surface into the tissue by selective cell enrichment in implants.

6. Implant, suitable for human and animal organs, consisting of a carrier matrix and a layer of a bioactive, cell adhesion-mediating molecule surrounding this matrix, **characterized in that** the surrounding layer is formed from a compound according to Claims 1 to 5, an ionic or adsorptive bond being present between carrier matrix and this compound.

7. Implant according to Claim 6, **characterized in that** the carrier matrix and/or its surface is a metal or a metal oxide.

8. Implant according to Claim 6, **characterized in that** the carrier matrix and/or its surface is a bone or tooth substitute material.

9. Implant according to Claim 8, **characterized in that** the bone or tooth substitute material consists of calcium phosphate mixtures.

10. Process for the preparation of a compound according to Claim 1, and of its salts, **characterized in that** a bioactive molecule B which can be provided with protective groups, and a spacer-anchor molecule ($Q-X_1$) or anchor molecule ($X_1$) provided with protective groups are linked to one another in peptide fashion and the protective groups are then removed, and/or **in that** a basic or acidic compound of the formula I is converted into one of its salts by treating with an acid or base.

11. Use of a compound according to one of Claims 1 to 4 for the production of a medicament for the treatment of metastases by inhibiting angiogenesis and the spread of tumour cells, for the antimicrobial and antiseptic treatment of inflammations and defects caused by the incorporation of implants and of osteolytic disorders such as osteoporosis, thrombosis, cardiac infarct and arteriosclerosis, and for the acceleration and strengthening of the integration process of implants or biocompatible surface into the tissue by selective cell enrichment in implants.

12. Use of a compound according to one of Claims 1 to 5 for the coating, by means of ionic or adsorptive binding, of implants for human and animal organs.

## Revendications

1. Composé de Formule 1

B-Q-X1

dans laquelle

B est une molécule bioactive se liant à l'intégrine,
Q manque ou est une molécule espaceur organique choisie dans le groupe

$[CO-(CH_2)_x-NH-]_m$ (xii)

$[CO-CH_2-(O-CH_2CH_2)_y-NH-]_m$ (xiii)

$[CO-(CH_2)_z-CO-]$ (xiv)

$[NH-(CH_2)_z-NH-]$ (xv)

$[CO-CH_2-(OCH_2CH_2)_y-O-CH_2-CO-]$ (xvi)

$[NH-CH_2CH_2-(OCH_2CH_2)_y-NH-]$ (xvii)

ainsi que leurs combinaisons,
où
chaque indice m vaut indépendamment des autres 1 à 20,

x vaut 1 à 12,
y vaut 1 à 50,
z vaut 1 à 12,

$X_1$ est une molécule d'ancrage choisie dans le groupe

$$-Lys-(CO-CH_2-(CH_2)_n-PO_3H_2)_2 \quad (i)$$

$$-Lys-[Lys-(CO-CH_2-(CH_2)_n-PO_3H_2)_2]_2 \quad (ii)$$

ou

$$-Lys-(Lys[-Lys-(CO-CH_2-(CH_2)_n-PO_3H_2)_2]_2 \quad (iii)$$

et chaque indice n vaut indépendamment des autres 0, 1, 2 ou 3, et où un groupe amino libre du groupe B est lié à un groupe carboxyle libre de la molécule espaceur Q ou de la molécule d'ancrage $X_1$, ou encore un groupe amino libre du radical Q est lié à un groupe carboxyle libre du radical $X_1$ à la manière d'une liaison peptidique, ainsi que ses sels.

**2.** Composé selon la revendication 1, dans lequel le groupe B est choisi dans le groupe

cyclo-(Arg-Gly-Asp-$Z_1$) (iv)

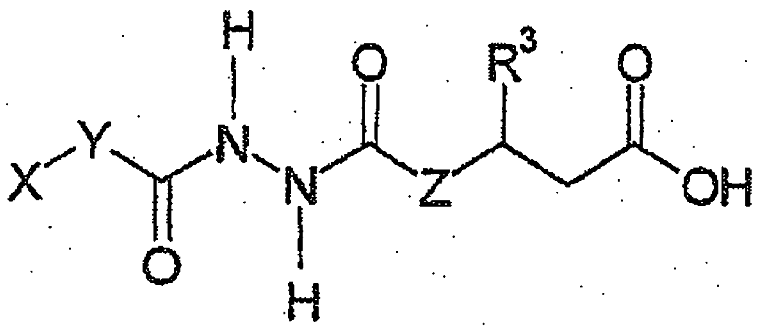

(V)

et

Thr-Trp-Tyr-Lys-Ile-Ala-Phe-Gln-Arg-Asn-Arg-Lys (vi)

Trp-Tyr-Lys-Ile-Ala-Phe-Gln-Arg-Asn-Arg-Lys (vii)

Tyr-Lys-Ile-Ala-Phe-Gln-Arg-Asn-Arg-Lys (viii)

Thr-Trp-Tyr-Lys-Ile-Ala-Phe-Gln-Arg-Asn-Arg (ix)

Thr-Trp-Tyr-Lys-Ile-Ala-Phe-Gln-Arg-Asn (x)

Thr-Trp-Tyr-Lys-Ile-Ala-Phe-Gln-Arg (xi)

où
chaque radical $Z_1$ représente indépendamment des autres un résidu d'acide aminé ou un résidu di- ou tripeptidique, les acides aminés étant choisis indépendamment les uns des autres dans un groupe consistant en Ala, Asn, Asp, Arg, Cys, Gln, Glu, Gly, His, Homo-Phe, Ile, Leu, Lys, Met, Orn, Phe, Phg, Pro, Ser, Thr, Trp, Tyr, Val,
où, pour (v)

X est $H_2N$-C(=NH)-NH, Het-NH-, $H_2N$-C(=NH)-, A-C(=NH)-NH ou Het-
Y est $-(CH_2)_n-$ ou

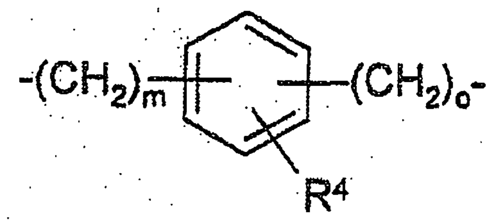

$-(CH_2)_s-CH(R^4)-(CH_2)_t-$ Ou $-(CH_2)_p-Het^1-(CH_2)_q-$,
Z est N-$R^2$ ou CH-$R^2$
$R^2$ est H ou un radical alkyle ayant 1 à 4 atomes de carbone,
$R^3$ est H, Ar, Het ou A,
$R^4$ est H, A, Ar, OH, OA, OAr, un radical arylalkyle, Hal, CN, $NO_2$, $CF_3$ ou $OCF_3$,
A est COOH, $NH_2$ ou un radical alkyle ayant 1 à 6 atomes de carbone, non substitué ou substitué par COOH ou $NH_2$,
Ar est un radical phényle non substitué ou substitué une fois, deux fois ou trois fois par A, OH, OA, $CF_3$, $OCF_3$, CN, $NO_2$ ou Hal, lequel radical phényle peut être substitué une fois, deux fois ou trois fois par un ou plusieurs radicaux phényle substitués par A, OH, OA, $NH_2$, $OCF_3$, CN, $NO_2$ ou Hal, de telle sorte qu'il se forme un biphényle non substitué ou substitué,
Hal est F, Cl, Br ou I,
Het est un radical hétérocyclique monocyclique ou bicyclique saturé, partiellement ou entièrement insaturé ayant 5 à 10 chaînons, où 1 à 3 atomes N et/ou 1 atome S ou O peuvent être présents, et le radical hétérocyclique peut être substitué une ou deux fois par CN, Hal, OH, $NH_2$, COOH, OA, $CF_3$, A, $NO_2$, Ar ou $OCF_3$,
$Het^1$ est un radical hétérocyclique aromatique à 5 ou 6 chaînons ayant 1 à 4 atomes N et/ou S, qui peut être non substitué, ou substitué une ou deux fois par F, Cl, Br, A, OA ou $OCF_3$,
n vaut 4, 5 ou 6
m, o, p, q valent 0, 1 ou 2
s, t valent 0, 1, 2, 3, 4 ou 5.

**3.** Composé selon la revendication 1 ou 2, dans lequel Q est choisi dans le groupe

$[CO-(CH_2)_x-NH-]_m$ (xviii)

$[CO-CH_2-(O-CH_2CH_2)_y-NH-]_m$ (xix)

$[CO-(CH_2)_2-CO-]$ (xx)

$[NH-(CH_2)_2-NH-]$ (xxi)

$[CO-CH_2-(OCH_2CH_2)_y-O-CH_2-CO-]$ (xxii)

$[NH-CH_2CH_2-(OCH_2CH_2)_y-NH-]$ (xxiii)

ainsi que leurs combinaisons,
où
chaque indice m vaut indépendamment des autres 1 à 8,

x vaut 1 à 5,
y vaut 1 à 6, et
z vaut 1 à 6.

**4.** Composés de formule 1 selon la revendication 1

a) Cyclo-(Arg-Gly-Asp-DPhe-Lys($N^{\varepsilon}H$-[CO-$(CH_2)_5$-NH]$_2$-Lys-[Lys-(CO-$CH_2$-$(CH_2)_n$-$PO_3H_2)_2]_2$)) ;
b) Cyclo-(Arg-Gly-Asp-DPhe-Lys($N^{\varepsilon}H$-[CO-$(CH_2)_5$-NH]$_3$-Lys-[Lys-(CO-$CH_2$-$(CH_2)_n$-$PO_3H_2)_2]_2$)) ;
c) Cyclo-(Arg-Gly-Asp-DPhe-Lys($N^{\varepsilon}H$-[CO-$CH_2$(-O-$CH_2CH_2)_6$-NH]$_2$-Lys[Lys-(CO-$CH_2$-$(CH_2)_n$-$PO_3H_2)_2]_2$)) ;

dans laquelle n vaut 1.

**5.** Composé selon l'une des revendications 1 à 4 pour utilisation en tant que médicament destiné au traitement de métastases par inhibition de l'angiogenèse et de la propagation de cellules tumorales, au traitement antimicrobien et antiseptique d'inflammations et de défauts provoqués par l'incorporation d'implants, et de maladies ostéolytiques telles que l'ostéoporose, la thrombose, l'infarctus du myocarde et l'artériosclérose, ainsi qu'à l'accélération et au renforcement du processus d'intégration d'implants ou de surfaces biocompatibles dans le tissu par un enrichissement cellulaire sélectif dans les implants.

**6.** Implant convenant à des organes humains ou animaux, constitué d'une matrice formant support et d'une couche enveloppant cette matrice d'une molécule bioactive favorisant l'adhésion cellulaire, **caractérisé en ce que** la couche enveloppante est formée d'un composé selon les revendications 1 à 5, une liaison ionique ou par adsorption étant présente entre cette matrice formant support et ce composé.

**7.** Implant selon la revendication 6, **caractérisé en ce que** la matrice formant support et/ou sa surface est un métal ou un oxyde métallique.

**8.** Implant selon la revendication 6, **caractérisé en ce que** la matrice formant support et/ou sa surface est un matériau pour prothèse osseuse ou dentaire.

**9.** Implant selon la revendication 8, **caractérisé en ce que** le matériau pour prothèse osseuse ou dentaire est constitué de mélanges de phosphate de calcium.

**10.** Procédé de préparation d'un composé selon la revendication 1, ainsi que de ses sels, **caractérisé en ce qu'**on assure une liaison peptidique entre une molécule bioactive B qui peut être pourvue de groupes protecteurs et une molécule espaceur-ancrage (Q-$X_1$) pourvue de groupes protecteurs ou une molécule d'ancrage ($X_1$), puis on clive les groupes protecteurs, et/ou on convertit en l'un de ses sels un composé basique ou acide de formule 1 par traitement avec un acide ou une base.

**11.** Utilisation d'un composé selon l'une des revendications 1 à 4 pour préparer un médicament destiné au traitement de métastases par inhibition de l'angiogenèse et de la propagation de cellules tumorales, au traitement antimicrobien et antiseptique d'inflammations et de défauts provoqués par l'incorporation d'implants, et de maladies ostéolytiques telles que l'ostéoporose, la thrombose, l'infarctus du myocarde et l'artériosclérose, ainsi qu'à l'accélération et au renforcement du processus d'intégration d'implants ou de surfaces biocompatibles dans le tissu par un enrichissement cellulaire sélectif dans les implants.

**12.** Utilisation d'un composé selon l'une des revendications 1 à 5 pour revêtir par liaison ionique ou par adsorption des implants destinés à des organes humains ou animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19932796
- DE 19755800
- DE 19831710
- DE 19755801
- DE 4310643
- EP 01960612 A


### In der Beschreibung aufgeführte Nicht-Patentliteratur


- **J.W. SMITH et al.** *J. Biol. Chem.,* 1990, vol. 265, 12267-12271
- **P.C. BROOKS ; R.A. CLARK ; D.A. CHERESH.** *Science,* 1994, vol. 264, 569-71
- **P.C. BROOKS ; A.M. MONTGOMERY ; M. ROSENFELD ; R.A. REISFELD ; T.-HU ; G. KLIER ; D.A. CHERESH.** *Cell,* 1994, vol. 79, 1157-64
- **B.C.F CHU et al.** Presentations of Ligands on Hydroxylapatite. *Bioconjugate Chemistry,* 1997, vol. 8, 103-105
- **P.VALENTIN-WEIGUND et al.** *Infection and Immunity,* 1988, 2851-2855
- **R.KEENAN et al.** *Abstr. Pap. 211th ACS National Meeting,* 1996
- **HOUBEN-WEYL, L.C.** LIEBIGS ANN. CHEM. 1974, vol. 15, 1-806
- **S. ZIMMER ; E. HOFFMANN ; G. JUNG ; H. KESSLER.** *Liebigs Ann. Chem.,* 1993, 497-501
- **R.B. MERRIFIELD.** *Angew. Chemie,* 1985, vol. 97, 801-812
- **B. F. GYSIN ; R. B. MERRIFIELD.** *J. Am. Chem. Soc.,* 1972, vol. 94, 3102
- **G.B. FIELDS ; R. L. NOBIE.** *Int. J. Pept. Protein Res.,* 1990, vol. 35, 161-214

SEQUENCE LISTING

<110> Biomet Deutschland GmbH

<120> Peptid- und Peptidmimetikakonjugate mit Integrin-Inhibitor-Eigenschaften

<130> BMM261EP

<140> EP 01960612
<141> 2001-08-02

<160> 9

<170> PatentIn version 3.1

<210> 1
<211> 12
<212> PRT
<213> Artificial Sequence

<220>
<223> Peptide
<220>
<221> PEPTIDE
<222> (1)..(12)
<223> peptide

<400> 1

Thr Trp Tyr Lys Ile Ala Phe Gln Arg Asn Arg Lys
1 5 10

<210> 2
<211> 11
<212> PRT
<213> Artificial Sequence

<220>
<223> Peptide
<220>
<221> PEPTIDE
<222> (1)..(11)
<223> peptide

<400> 2

Trp Tyr Lys Ile Ala Phe Gln Arg Asn Arg Lys
1 5 10

<210> 3
<211> 10
<212> PRT
<213> Artificial Sequence
<220>
<223> Peptide
<220>
<221> PEPTIDE
<222> (1)..(10)
<223> peptide

<400> 3

Tyr Lys Ile Ala Phe Gln Arg Asn Arg Lys
1 5 10

<210> 4
<211> 11
<212> PRT
<213> Artificial Sequence

<220>
<223> Peptide
<220>
<221> PEPTIDE
<222> (1)..(11)
<223> peptide

<400> 4

Thr Trp Tyr Lys Ile Ala Phe Gln Arg Asn Arg
1 5 10

<210> 5
<211> 10
<212> PRT .
<213> Artificial sequence

<220>
<223> Peptide
<220>
<221> PEPTIDE
<222> (1)..(10)
<223> peptide

<400> 5

Thr Trp Tyr Lys Ile Ala Phe Gln Arg Asn
1 5 10

<210> 6
<211> 9
<212> PRT
<213> Artificial Sequence

<220>
<223> Peptide
<220>
<221> PEPTIDE
<222> (1)..(9)
<223> peptide

<400> 6

Thr Trp Tyr Lys Ile Ala Phe Gln Arg
1 5

<210> 7
<211> 5
<212> PRT
<213> Artificial Sequence

<220>
<223> Peptide
<220>
<221> misc_feature
<223> cyclopentapeptide

<220>
<221> misc_feature
<222> (4)..(4)
<223> Xaa at position 4 is D-Phe

<220>
<221> misc_feature
<222> (5)..(5)
<223> xaa at position 5 is Lys(NH-[CO-(CH2)5-NH]2-Lys-[Lys(CO-CH2CH2PO3H2 )2)2))

<400> 7

Arg Gly Asp Xaa Xaa
1 5

<210> 8
<211> 5
<212> PRT
<213> Artificial Sequence

<220>
<223> Peptide

<220>
<221> misc_feature
<222> (1)..(5)
<223> cyclopentapeptide

<220>
<221> misc_feature
<222> (4)..(4)
<223> xaa at position 4 is D-Phe

<220>
<221> misc_feature
<222> (5)..(5)
<223> xaa at position 5 is Lys(NH-[CO-(CH2)5-NH]3-Lys-[Lys-(CO-CH2CH2PO3H2)2)2 ))

<400> 8

Arg Gly Asp Xaa Xaa
1 5

<210> 9
<211> 5
<212> PRT
<213> Artificial sequence

<220>
<223> Peptide
<220>
<221> misc_feature
<222> (1)..(5)
<223> cyclopentapeptide

<220>
<221> misc_feature
<222> (4)..(4)
<223> xaa at position 4 is D-Phe

<220>
<221> misc_feature
<222> (5)..(5)
<223> Xaa at position 5 is
Lys(NH-[CO-CH2(-O-CH2CH2)6-NH]2-Lys-[Lys-(CO-CH2CH2PO3H2 )2)2))

<400> 9

Arg Gly Asp Xaa Xaa
1 5